# EUROPEAN PATENT SPECIFICATION

(11) **EP 3 583 417 B1**
(45) Date of publication and mention of the grant of the patent: **12.05.2021**
(21) Application number: 18707133.7
(22) Date of filing: 16.02.2018
(51) Int. Cl.: G01N 33/12, G01N 33/74, H01J 49/04

(54) **ANALYSIS OF FOOD SAMPLES**
ANALYSE VON LEBENSMITTELPROBEN
ANALYSE D'ÉCHANTILLONS ALIMENTAIRES

(30) Priority: 17.02.2017 GB 201702643
(43) Date of publication of application: 25.12.2019
(73) Proprietor: Micromass UK Limited, Wilmslow SK9 4AX (GB)
(72) Inventor: STEAD, Sara Lucy, Congleton, Cheshire CW12 2HF (GB); JANDOVA, Renata, Wilmslow, Cheshire SK9 3LW (GB)
(74) Representative: Dehns
(86) International application number: PCT/GB2018/050419
(87) International publication number: WO 2018/150193

(56) References cited:
- EP-A1- 1 233 267
- JULIA BALOG ET AL: "Identification of the Species of Origin for Meat Products by Rapid Evaporative Ionization Mass Spectrometry", JOURNAL OF AGRICULTURAL AND FOOD CHEMISTRY, vol. 64, no. 23, 11 May 2016 (2016-05-11), pages 4793-4800, XP55468335, US ISSN: 0021-8561, DOI: 10.1021/acs.jafc.6b01041
- NIELEN M W F ET AL: "Desorption electrospray ionization mass spectrometry in the analysis of chemical food contaminants in food", TRAC TRENDS IN ANALYTICAL CHEMISTRY, vol. 30, no. 2, 9 December 2010 (2010-12-09), pages 165-180, XP028361909, ISSN: 0165-9936, DOI: 10.1016/J.TRAC.2010.11.006 [retrieved on 2010-12-09]
- JACK J. LOHNE ET AL: "Laser diode thermal desorption mass spectrometry for the analysis of quinolone antibiotic residues in aquacultured seafood", RAPID COMMUNICATIONS IN MASS SPECTROMETRY., vol. 26, no. 24, 5 November 2012 (2012-11-05), pages 2854-2864, XP55272575, GB ISSN: 0951-4198, DOI: 10.1002/rcm.6414
- VERPLANKEN KAAT ET AL: "Rapid evaporative ionization mass spectrometry for high-throughput screening in food analysis: The case of boar taint", TALANTA, vol. 169, 21 March 2017 (2017-03-21), pages 30-36, XP029978556, ISSN: 0039-9140, DOI: 10.1016/J.TALANTA.2017.03.056

## Description

### CROSS-REFERENCE TO RELATED APPLICATION

This application claims priority from and the benefit of United Kingdom patent application No. 1702643.6 filed on 17 February 2017.

### FIELD OF THE INVENTION

The present invention relates generally to mass and/or ion mobility spectrometers and in particular to mass and/or ion mobility spectrometric analysis of food samples.

### BACKGROUND

During the past decades, public awareness of food safety and quality has significantly increased. The organoleptic properties of food, e.g. its taste and smell, play a crucial part in this, as they are reflective of the first impressions consumers will develop.

One particularly important example is in the meat industry. Boar taint is an off-odour that can be present in porcine meat (pork) from uncastrated male pigs. Porcine meat which has come from an animal suffering from boar taint can be unattractive to consumers, as the smell can put the consumer off eating the meat in question, and even from buying more porcine meat.

The surgical castration of pigs has been used to prevent boar taint. However, increasing awareness of animal welfare has led to the voluntarily abandonment of surgical castration of male pigs in many countries.

Consequently, there is a need to test food for its quality, including in particular for its organoleptic properties, such as for example for the presence or absence of boar taint in pork.

Presently, sensory methods are often used to test food for its quality. For example, a soldering iron may be applied to the fatty tissue of an animal carcass and the released smell may be assessed by a trained assessor. Such sensory methods can be imprecise and expensive, since for example, they rely on the sensory abilities of one trained assessor and can be subject to inter-individual variation, habituation and fatigue.

The various analytical methods that have been proposed to date for this purpose often lack sensitivity, specificity and/or high-throughput.

For example, Raman spectrometry based approaches for detecting boar taint have been shown to produce accurate results, but require an analysis time of about 20 minutes per sample, which is prohibitive in a commercial setting.

Similarly, Gas Chromatography Mass Spectrometric (GCMS) approaches for detecting boar taint are not sufficiently rapid, and also are not sensitive enough.

It will be appreciated, therefore, that there is a need for improved techniques for analysing food.

Balog et al. in Journal of Agricultural and Food Chemistry 64 (2016), pages 4793-4800, discloses analysing a food sample, wherein ions are generated from the food sample by ambient ionisation, the ions are analysed to produce a data set and assessed in an untargeted analysis to determine properties of the food sample.

EP1233267 discloses detecting boar taint of a sample provided on a slaughter line.

### SUMMARY

According to an aspect there is provided a method of analysing a food sample as defined in claim 1. In accordance with the present invention, the method determines one or more properties comprising one or more organoleptic properties such as taste and/or smell.

The Applicants have recognised that ambient ionisation techniques are particularly suited to the analysis of food samples, such as for example, the detection of boar taint in porcine meat (pork). In particular, and as will be described in more detail below, the Applicants have demonstrated that such techniques are sufficiently inexpensive, reproducible, sensitive, specific, and rapid to be implemented in a commercial setting.

The ambient ionisation techniques according to various embodiments can in particular be used for in-situ analysis of animal carcasses on a slaughter line. Many hundreds of animal carcasses may be processed per hour on a slaughter line, e.g. an average of around 600 pig carcasses may be processed per hour on a pig slaughter line. This represents a significant challenge for existing analysis techniques.

Ambient ionisation techniques such as rapid evaporative ionisation mass spectrometry (REIMS) techniques circumvent long analysis times by enabling direct ionisation from the sample. As such, ambient ionisation analysis according to various embodiments takes only a few seconds. Such techniques can also be automated to guarantee point-of-control analysis. Additionally, ambient ionisation analysis such as REIMS analysis can provide highly accurate identification of samples.

It will be appreciated, therefore, that various embodiments provide an improved method of analysing food samples.

The step of assessing the data set to determine the one or more properties of the food sample comprises using an untargeted analysis technique.

The Applicants have recognised that untargeted analysis techniques are particularly suited for assessing organoleptic properties of the food samples.

The step of using an untargeted analysis technique comprises using a predictive model to classify the sample into one of two or more groups.

When the sample is identified or determined to have one or more particular properties, then the method may further comprise:
associating with the sample information indicating that the sample has the one or more particular properties; and/or
separating the sample from one or more other samples. The sample may be provided on a production line or a slaughter line.

The Applicants have recognised that various embodiments are particularly suited to the analysis of animal carcasses on a slaughter line. For example, if it is determined that a particular carcass has one or more undesirable properties such as boar taint, then that carcass may be labelled as such and/or may be separated from other carcasses on the slaughter line.

The food sample may comprise an edible product of animal origin including a meat or fish sample.

The meat sample may comprise a porcine meat (pork) sample.

The step of assessing the data set to identify or determine the one or more properties of the food sample may comprise assessing the data set to determine whether boar taint is present in the porcine meat sample.

The Applicants have recognised that various embodiments are particularly suited to the detection of boar taint in porcine meat (pork) samples.

The ambient ionisation system may comprise a sampling probe, and the method may comprise using the sampling probe to generate aerosol, smoke, vapour or droplets and/or the analyte ions from the sample.

The method may comprise:
sensing the presence and/or location of the sample; and then automatically causing the sampling probe to interact with the sample; and/or
automatically moving the sampling probe into contact or engagement with the sample; and/or
automatically activating or energising the sampling probe.

The method of may comprise causing the sampling probe to interact with the sample, contacting or engaging the sampling probe with the sample and/or activating the sampling probe for only a few seconds or a few tens of seconds.

Using ambient ionisation generate analyte ions from the sample may comprise applying an electric current to the sample.

Using ambient ionisation to generate analyte ions from the sample may comprise directing a laser beam onto the sample.

Using ambient ionisation to generate analyte ions from the sample may comprise directing a spray of charged droplets onto the sample.

Using ambient ionisation to generate analyte ions from the sample may comprise using ambient ionisation in negative ionisation mode.

According to an aspect, there is provided an analytical instrument for analysing a food sample as defined in claim 14.

The analytical instrument may be configured to: when the sample is identified or determined to have the one or more particular properties:
associate with the sample information indicating that the sample has the one or more particular properties; and/or
separate the sample from one or more other samples.

The sample may be provided on a production line or a slaughter line.

According to an aspect, there is provided a production line or a slaughter line as defined in claim 15.

In accordance with the present invention, the analytical instrument is configured to determine one or more properties comprising one or more organoleptic properties of the food sample.

The food sample may comprise an edible product of animal origin including a meat or fish sample.

The meat sample may comprise a porcine meat (pork) sample.

The step of assessing the data set to identify or determine one or more properties of the food sample may comprise assessing the data set to determine whether boar taint is present in the porcine meat sample.

The ambient ionisation system may comprise a sampling probe configured to generate aerosol, smoke, vapour or droplets and/or the analyte ions from the sample.

The ambient ionisation system may comprise an ambient ionisation source configured to generate analyte ions, e.g. from the aerosol, smoke, vapour or droplets.

The analytical instrument or the production line or the slaughter line may comprise one or more sensors configured to sense the presence and/or location of the sample.

The analytical instrument or the production line or the slaughter line may be configured to automatically cause the sampling probe to interact with the sample, move the sampling probe into contact or engagement with the sample, and/or activate or energise the sampling probe.

The analytical instrument or the production line or the slaughter line may be configured to cause the sampling probe to interact with the sample, contact or engage the sampling probe with the sample and/or to activate the sampling probe for only a few seconds or a few tens of seconds.

The ambient ionisation system may be configured to apply an electric current to the sample.

The ambient ionisation system may be configured to direct a laser beam onto the sample.

The ambient ionisation system may be configured to direct a spray of charged droplets onto the sample.

The ambient ionisation system may be configured to operate in negative ionisation mode.

The sample may be a meat sample.

The meat may be porcine.

The step of identifying the quality of the sample may comprise determining the presence or absence of boar taint.

The ambient ion source may comprise a laser.

The ambient ion source may comprise an electric current.

The spectrometer may be operated in various modes of operation including a mass spectrometry ("MS") mode of operation; a tandem mass spectrometry ("MS/MS") mode of operation; a mode of operation in which parent or precursor ions are alternatively fragmented or reacted so as to produce fragment or product ions, and not fragmented or reacted or fragmented or reacted to a lesser degree; a Multiple Reaction Monitoring ("MRM") mode of operation; a Data Dependent Analysis ("DDA") mode of operation; a Data Independent Analysis ("DIA") mode of operation a Quantification mode of operation or an Ion Mobility Spectrometry ("IMS") mode of operation.

### BRIEF DESCRIPTION OF THE DRAWINGS

Various embodiments will now be described, by way of example only, and with reference to the accompanying drawings in which:
Fig. 1 shows an analytical instrument according to an embodiment;
Fig. 2 shows schematically the rapid evaporative ionisation mass spectrometry (REIMS) technique according to an embodiment;
Fig. 3 shows schematically the desorption electrospray ionisation (DESI) technique according to an embodiment;
Fig. 4 shows a score plot of a partial least-squares discriminant analysis model for a dataset containing blank (sow) (n=50), negative (untainted) (n=50) and positive (tainted) (n=50) boar neck fat samples in negative ionisation mode;
Fig. 5 shows a score plot of a partial least-squares discriminant analysis model for a dataset containing negative (untainted) (n=50) and positive (tainted) (n=50) boar neck fat samples in negative ionisation mode;
Fig. 6 shows mass spectral fingerprints for negative (untainted (n=50), positive (tainted) (n=50) boar and blank (sow) (n=50) neck fat samples obtained in A) negative and B) positive ionisation mode;
Fig. 7 shows a heat map visualizing a selected number of putatively identified compounds in blank (sow) (n=50), positive (tainted) (n=50) and negative (untainted) (n=50) neck fat samples, with hierarchical clustering of the different samples;
Fig. 8 shows a principal component analysis (PCA) plot of a data set comprising regular beef, Welsh premium beef and buffalo samples; and
Fig. 9 shows a linear discriminant analysis (LDAA) plot of a data set comprising regular beef, Welsh premium beef and buffalo samples.

### DETAILED DESCRIPTION

In a first aspect, the invention is directed to a method of analysing a food sample, as defined in claim 1.

The food sample may comprise any suitable food sample. In particular, the food sample may comprise an edible product of animal origin including a meat or fish sample, such as for example a porcine meat (pork) sample. However, it would be possible to analyse other food, meat and fish samples using the techniques described herein.

The sample may be part of or derived from a food product such as an animal carcass. In particular, the sample may be part of or derived from a male pig, e.g. an uncastrated male pig.

The sample may be analysed directly, e.g. without (signification) sample preparation, and under ambient conditions (e.g. atmospheric conditions).

The food product may be provided on a production line, or in the case of meat products (e.g. animal carcasses), on a slaughter line, and the analysis may be performed with the product in-situ on the line. As such, the present invention can facilitate at-line detection of organoleptic properties such as boar taint.

Many hundreds of animal carcasses may be processed per hour on a slaughter line, e.g. an average of around 600 pig carcasses may be processed per hour on a pig slaughter line. This represents a significant challenge for existing analysis techniques. The ambient ionisation techniques circumvent long analysis times by enabling direct ionisation from the sample. The ambient ionisation analysis takes only a few seconds.

According to various embodiments, the ambient ionisation system comprises a sampling probe. The sampling probe may be configured to interact with, e.g. provide energy to, the sample. The sampling probe may be configured to be brought into contact or otherwise engaged with (e.g. brought into close proximity with) the sample. The sampling probe may be configured to be activated (e.g. turned on) to generate analyte ions from the sample. The sampling probe may be connected to an analytical instrument via one or more transfer tubes, e.g. wherein the analyte ions generated by the sampling probe are transferred to the analytical instrument via the one or more transfer tubes.

According to these embodiments, the sampling probe is brought into contact or otherwise caused to interact with or engaged with the sample and/or activated (in order to generate analyte ions from the sample) for a relatively short period of time. For example, the sampling probe may be brought into contact or otherwise caused to interact with or engaged with the sample and/or activated (in order to generate analyte ions from the sample) for time of only a few seconds or
only a few tens of seconds. The sampling probe may be brought into contact or otherwise caused to interact with or engaged with the sample and/or activated (in order to generate analyte ions from the sample) for (i) < 30s; (ii) < 25 s; (iii) < 20 s; (iv) < 15 s; (v) < 10 s; (vi) < 5 s; (vii) < 3 s; or (viii) < 1 s.

As such, the technique may be used to assess (i) > 300 samples per hour; (ii) > 400 samples per hour; (iii) > 600 samples per hour; (iv) > 800 samples per hour; or (v) > 1000 samples per hour.

The sampling probe may comprise a user operated sampling probe such as a handheld sampling probe, e.g. wherein the sampling probe may be configured to be brought into contact or otherwise caused to interact with or engaged with the sample and/or activated by a user.

Alternatively, the sampling probe may comprise an automated sampling probe, e.g. which requires little or no user interaction. In these embodiments, the sampling probe may be configured to be automatically brought into contact or otherwise caused to interact with or engaged with the sample and/or automatically activated so as to generate analyte ions from the sample.

One or more sensors may be provided that are configured to determine the presence (or absence) and/or location of a sample (or the presence (or absence) and/or location of a product of which the sample is part) to be analysed. The one or more sensors may comprise, for example, one or more (e.g. mechanical) sensors configured to determine the presence of a sample (product) when its weight or another force caused by the sample (product) is detected. It would also or instead be possible for the one or more sensors to utilise, for example, image recognition techniques, etc.

According to these embodiments, when the presence of a sample (product) is detected by the one or more sensors, then the sampling probe may be automatically brought into contact or otherwise caused to interact with (e.g. to provide energy to) or engaged with the sample and/or automatically activated so as to generate analyte ions from the sample.

The ambient ionisation ion system may comprise an automated sampling probe that is integrated into the production line or slaughter line. In these embodiments, the presence of one or more or each food product, e.g. animal carcass, on the production line or slaughter line may be detected by one or more sensors, e.g. as described above. The automated sampling probe may then be automatically (e.g. robotically) brought into contact or otherwise caused to interact with or engaged with the sample and/or automatically activated, e.g. so as to generate analyte ions from the food product in question.

It will be appreciated that these automated techniques can be configured to guarantee point-of-control analysis.

The ambient ionisation system may comprise any suitable ambient ionisation ion system. Ambient ionisation ion systems are capable of generating gas-phase ions directly from native (i.e. untreated or unmodified) samples. A particular benefit of ambient ionisation techniques is that they do not require any prior sample preparation.

The ambient ion system may comprise a rapid evaporative ionisation mass spectrometry (REIMS) ion system.

In these embodiments, an electric current may be used to generate an aerosol, smoke, vapour or droplets (droplet stream) from the sample. A sampling probe may be brought into contact (or into close proximity) with the sample and the sampling probe may be activated by applying an RF voltage to the sampling probe, e.g. to cause localised Joule or diathermy heating of the sample. As a result, aerosol, smoke, vapour or droplets may be generated.

Additionally or alternatively, a laser beam may be used to generate an aerosol, smoke, vapour or droplets (droplet stream) and/or analyte ions from the sample. In this case, the sampling probe may comprise a laser. The sampling probe may be activated by turning on the laser. The sampling probe may be engaged with the sample by directing the laser beam (produced by the laser) onto the sample, e.g. so as to generate aerosol, smoke, vapour or droplets and/or analyte ions from the sample.

The aerosol, smoke, vapour or droplets and/or analyte ions may then be transferred to an analytical instrument such as a mass and/or ion mobility spectrometer, e.g. via its atmospheric pressure interface.

A matrix comprising an organic solvent such as isopropanol may be added to the aerosol, smoke, vapour or droplets, e.g. at or prior to the atmospheric pressure interface of the analytical instrument. The mixture of aerosol, smoke, vapour or droplets and organic solvent may then be arranged to impact upon a collision surface. The collision surface may be located within a vacuum chamber of the analytical instrument. Alternatively, the collision surface may be arranged external to the analytical instrument, e.g. close to the inlet of the analytical instrument.

The aerosol, smoke, vapour or droplets may be caused to ionise upon impacting the collision surface resulting in the generation of analyte ions. The ionisation efficiency of generating the analyte ions may be improved by the addition of the organic solvent. However, the addition of an organic solvent is not essential.

The collision surface may comprise any suitable such collision surface. The collision surface may be formed from any suitable material, such as a metal or a ceramic. The collision surface may be heated. This can improve the ionisation efficiency.

According to other embodiments, the ambient ion system may comprise a desorption electrospray Ionisation (DESI) ion system. The desorption electrospray ionisation (DESI) technique allows for ambient ionisation of a trace sample at atmospheric pressure with little sample preparation.

In these embodiments, a spray of (primary) electrically charged droplets may be directed onto the surface of the sample. The droplets may be generated by a sampling probe in the form of a sprayer device such as a nebuliser. Subsequent ejected (e.g. splashed) (secondary) droplets may carry desorbed ionised analytes (e.g. desorbed lipid ions).

The sprayer device may be supplied with a solvent, nebulising gas such as nitrogen, and voltage from a high voltage (HV) source. The solvent may be supplied to a central capillary of the sprayer, and the nebulising gas may be supplied to a second capillary that may (at least partially) coaxially surround the central capillary. The arrangement of the capillaries, the flow rate of the solvent and/or the flow rate of the gas may be configured such that solvent droplets are ejected from the sprayer. The high voltage may be applied to the central capillary, e.g. such that the ejected solvent droplets are charged.

The charged droplets may be directed at the sample such that subsequent ejected (secondary) droplets carry desorbed analyte ions. The ions may travel into an atmospheric pressure interface of an analytical instrument such as a mass and/or ion mobility spectrometer, e.g. via a transfer capillary.
It would also be possible to use other ionisation techniques. For example, the ion system may comprise (i) a rapid evaporative ionisation mass spectrometry ("REIMS") ion source; (ii) a desorption electrospray ionisation ("DESI") ion source; (iii) a laser desorption ionisation ("LDI") ion source; (iv) a thermal desorption ion source; (v) a laser diode thermal desorption ("LDTD") ion source; (vi) a desorption electro-flow focusing ("DEFFI") ion source; (vii) a dielectric barrier discharge ("DBD") plasma ion source; (viii) an Atmospheric Solids Analysis Probe ("ASAP") ion source; (ix) an ultrasonic assisted spray ionisation ion source; (x) an easy ambient sonic-spray ionisation ("EASI") ion source; (xi) a desorption atmospheric pressure photoionisation ("DAPPI") ion source; (xii) a paperspray ("PS") ion source; (xiii) a jet desorption ionisation ("JeDI") ion source; (xiv) a touch spray ("TS") ion source; (xv) a nano-DESI ion source; (xvi) a laser ablation electrospray ("LAESI") ion source; (xvii) a direct analysis in real time ("DART") ion source; (xviii) a probe electrospray ionisation ("PESI") ion source; (xix) a solid-probe assisted electrospray ionisation ("SPA-ESI") ion source; (xx) a cavitron ultrasonic surgical aspirator ("CUSA") device; (xxi) a focussed or unfocussed ultrasonic ablation device; (xxii) a microwave resonance device; or (xxiii) a pulsed plasma RF dissection device.

The ambient ionisation ion system may be operated in negative ion mode or positive ion mode. However, the Applicants have found that better classification accuracy can be achieved using negative ionisation mode. Thus, generating analyte ions from the sample using ambient ionisation may comprise using ambient ionisation in negative ionisation mode.

Analyte ions generated by the ambient ionisation source may be passed through subsequent stages of the analytical instrument, and e.g. subjected to one or more of: separation and/or filtering using a separation and/or filtering device, fragmentation or reaction using a collision, reaction or fragmentation device, and analysis using an analyser.

The analyte ions may be (directly) analysed, and/or ions derived from the analyte ions may be analysed. For example, some or all of the analyte ions may be fragmented or reacted so as to produce product ions, e.g. using a collision, reaction or fragmentation device, and these product ions (or ions derived from these product ions) may then be analysed.

Suitable collision, fragmentation or reaction cells include, for example: (i) a Collisional Induced Dissociation ("CID") fragmentation device; (ii) a Surface Induced Dissociation ("SID") fragmentation device; (iii) an Electron Transfer Dissociation ("ETD") fragmentation device; (iv) an Electron Capture Dissociation ("ECD") fragmentation device; (v) an Electron Collision or Impact Dissociation fragmentation device; (vi) a Photo Induced Dissociation ("PID") fragmentation device; (vii) a Laser Induced Dissociation fragmentation device; (viii) an infrared radiation induced dissociation device; (ix) an ultraviolet radiation induced dissociation device; (x) a nozzle-skimmer interface fragmentation device; (xi) an in-source fragmentation device; (xii) an in-source Collision Induced Dissociation fragmentation device; (xiii) a thermal or temperature source fragmentation device; (xiv) an electric field induced fragmentation device; (xv) a magnetic field induced fragmentation device; (xvi) an enzyme digestion or enzyme degradation fragmentation device; (xvii) an ion-ion reaction fragmentation device; (xviii) an ion-molecule reaction fragmentation device; (xix) an ion-atom reaction fragmentation device; (xx) an ion-metastable ion reaction fragmentation device; (xxi) an ion-metastable molecule reaction fragmentation device; (xxii) an ion-metastable atom reaction fragmentation device; (xxiii) an ion-ion reaction device for reacting ions to form adduct or product ions; (xxiv) an ion-molecule reaction device for reacting ions to form adduct or product ions; (xxv) an ion-atom reaction device for reacting ions to form adduct or product ions; (xxvi) an ion-metastable ion reaction device for reacting ions to form adduct or product ions; (xxvii) an ion-metastable molecule reaction device for reacting ions to form adduct or product ions; (xxviii) an ion-metastable atom reaction device for reacting ions to form adduct or product ions; and/or (xxix) an Electron lonisation Dissociation ("EID") fragmentation device.

Some or all of the analyte ions or ions derived from the analyte ions may be filtered, e.g. using a mass filter. Suitable mass filters include, for example: (i) a quadrupole mass filter; (ii) a 2D or linear quadrupole ion trap; (iii) a Paul or 3D quadrupole ion trap; (iv) a Penning ion trap; (v) an ion trap; (vi) a magnetic sector mass filter; (vii) a Time of Flight mass filter; and/or (viii) a Wien filter.

The analyte ions or ions derived from the analyte ions may be mass analysed, e.g. using a mass analyser, i.e. so as to determine their mass to charge ratio. As such, the data set may comprise one or more mass spectra.

Suitable mass analysers include, for example: (i) a quadrupole mass analyser; (ii) a 2D or linear quadrupole mass analyser; (iii) a Paul or 3D quadrupole mass analyser; (iv) a Penning trap mass analyser; (v) an ion trap mass analyser; (vi) a magnetic sector mass analyser; (vii) Ion Cyclotron Resonance ("ICR") mass analyser; (viii) a Fourier Transform Ion Cyclotron Resonance ("FTICR") mass analyser; (ix) an electrostatic mass analyser arranged to generate an electrostatic field having a quadro-logarithmic potential distribution; (x) a Fourier Transform electrostatic mass analyser; (xi) a Fourier Transform mass analyser; (xii) a Time of
Flight mass analyser; (xiii) an orthogonal acceleration Time of Flight mass analyser; and/or (xiv) a linear acceleration Time of Flight mass analyser.

Additionally or alternatively, the analyte ions or ions derived from the analyte ions may be analysed using an ion mobility separation device and/or a Field Asymmetric Ion Mobility Spectrometer (FAIMS) device. As such, the data set may comprise one or more ion mobility or FAIMS spectra.

The data set could also comprise one or more mass-to-charge ratio/ion mobility or FAIMS data sets.

The data set, e.g. mass and/or ion mobility spectrum or spectra, is assessed to identify the one or more properties of the food sample.

The one or more properties may include plural properties of the food sample. In accordance with the present invention, the one or more properties comprise one or more organoleptic, i.e. sensory, properties such as taste and/or smell. In one particular such embodiment, the organoleptic property is the presence or absence of boar taint. Thus, the method may comprise assessing the data set to determine the presence (or absence) of boar taint in the food sample. Additionally, the one or more properties may comprise the presence (or absence), quantity and/or identity of: (i) one or more microbes; (ii) one or more pathogens; (iii) one or more parasites or excreta or other waste products from parasites; and/or (iv) one or more fungi, in the food sample. For example, the data set may be assessed to determine the presence (or absence) or quantity of salmonella (e.g. in chicken), e-coli, etc. in the sample. Additionally, the one or more properties may comprise the (i) species, subspecies or other taxonomic classification; (ii) gender; (iii) health; (iv) age; and/or (v) geographical origin; of the animal from which the sample is derived.

For example, the data set may be assessed to determine whether (or not) the food sample is a particular, e.g. high value, food sample such as wagyu beef and the like. Additionally, the one or more properties may comprise one or more properties relating to the manner in which the animal from which the sample is derived was raised, produced, slaughtered and/or caught. For example, the one or more properties may be related to whether (or not) the animal was: (i) raised according to "organic" standards; (ii) free range (or battery raised); (iii) line caught or trawler caught (fish); and/or (iv) slaughtered or produced according to Halal or other standards. Such properties may be determined, e.g. by assessing the data set to determine the presence (or absence) and/or quantity of one or more relevant hormones (e.g. stress hormones), chemicals (e.g. synthetic chemicals such as fertilizers, pesticides, antibiotics, food additives), and the like.

The food sample may comprise an edible product of animal origin including a meat or fish (e.g. poultry) sample (such as for example a chicken meat sample, an egg, milk, or honey sample, etc.), and the one or more properties may additionally comprise one or more properties relating to the production method of the (meat or fish (e.g. poultry)) sample, e.g. relating to whether (or not) the (meat or fish (e.g. poultry)) sample was produced using (i) a high welfare production method; (ii) an organic production method; (iii) a standard production method; (iv) a particular feeding regime such as a corn fed production method; and the like.

The food sample may comprise an edible product of animal origin including a meat or fish sample, and the one or more properties may additionally comprise one or more properties relating to the quality of the (meat or fish) sample, such as the (degree or level of) tenderness of the meat sample (e.g. a degree or level of beef tenderness).

The food sample may comprise an edible product of animal origin including a meat or fish sample, and in an embodiment of the present invention, the one or more properties may comprise one or more properties relating to the flavour or taste of the (meat or fish) sample. For example, the one or more properties may relate to a meat (e.g. lamb) flavour grading.

The food sample may comprise an edible product of animal origin including a meat sample, and the one or more properties may additionally comprise one or more properties relating to a (meat) grading of the (meat) sample, e.g. relating to whether (or not) the (meat) sample (i) comprises a premium variety; (ii) comprises a standard variety; or (iii) was produced using a particular feeding regime.

The food sample may comprise an edible product of animal origin including a meat or fish sample, and the one or more properties may additionally comprise one or more properties relating to an animal stress level, e.g. one or more indirect animal stress markers, e.g. relating to (i) Pale, Soft, Exudative meat (PSE meat); (ii) dark cutting meat; and the like. For example, the data set may be assessed to determine the presence (or absence) or quantity of cortisol and/or perturbations in the hypothalamic pituitary adrenal (HPA) axis in the sample.

The food sample may comprise an edible product of animal origin including a meat or fish sample, and the one or more properties may additionally comprise one or more properties (e.g. indirect markers) relating to the presence of, absence of or exposure to one or more xenobiotics of such as (i) steroid hormones; (ii) beta-agonists; (iii) antibiotics; (iv) natural toxins; (v) contaminants; and/or (vi) beta-agonists; and the like.

It is believed that boar taint is caused by the build-up of Indole (IND), skatole (SK) and Androsterone (AEON) in the Adipose tissue of a boar. IND and SK are two indolic compounds derived from the biological degradation of L-tryptophan in the hindgut and their odour is often described as faecal like. AEON is a pheromone produced in the Leydig cells of the testis and has a urinary or sweaty odour.

According to the present invention, the data set is assessed using an untargeted analysis technique, because the Applicants have found that an untargeted method (i.e. untargeted mass spectrometry) is preferable to assess the data set, where the one or more properties comprise one or more organoleptic (sensory) properties such as boar taint.

In this regard, the Applicants have found that untargeted profiling provides a significantly improved accuracy in identifying organoleptic (sensory) properties such as boar taint, e.g. when compared with targeted approaches. This is because although certain compounds may be known to contribute significantly to organoleptic properties (such as Indole (IND), skatole (SK) and Androsterone (AEON) that contribute to boar taint), the cause of subjective sensory properties (such as boar taint) may be complex, e.g. may depend on other compounds, the particular balance of these compounds in the sample, etc. The Applicants have found that untargeted profiling techniques are much more suited to identifying organoleptic (sensory) properties in a sample, and that untargeted profiling approaches correlate closely with the known sensory analysis techniques (e.g. that use a trained assessor).

The analysis may take into account all of or at least a large proportion of the data set (rather than merely identifying one or more individual ions or interest in the data set). In particular, the analysis may take into account the (entire) mass spectral fingerprint (the entire data set).

A predictive model may be built using data sets acquired from samples known to have the one or more properties, e.g. boar taint, together with data sets acquired from samples known not to have the one or more properties. That is, a predictive model may be built using a so-called "training set" of data sets. The model may comprise, for example, a principal component analysis (PCA) model such as an orthogonal partial least-square discriminant analysis (OPLS-DA) model, or a linear discriminant analysis (LDA) model, and the like.

The model is used to classify a sample into one of two or more groups based on the data set, e.g. based on the mass spectrometric fingerprint of the sample. The two or more groups may comprise, for example, a positive group indicating that the sample has the one or more properties (e.g. boar taint), and a negative group indicating that the sample does not have (other than has) the one or more properties (e.g. boar taint).

Thus, the step of assessing the data set to identify one or more properties of the food sample comprises using a predictive model, e.g. an OPLS-DA model, to classify the sample into one of two or more groups based on the (entire) data set, e.g. based on the (entire) mass spectrometric fingerprint of the sample.

The sample and/or the data set may be classified using any one or more of the techniques described in WO2016/142692 (Micromass).

When it is determined that a food sample has the one or more properties and/or one or more particular properties of interest, then information indicating that the sample or product has the one or more (particular) properties may be associated with the sample or product.

Correspondingly, when it is determined that a food sample does not have (other than has) the one or more properties and/or one or more particular properties of interest, then information indicating that the sample or product does not have (other than has) the one or more (particular) properties may be associated with the sample or product.

The information may be associated with the sample or product in any suitable manner. For example, the information may be physically associated with the sample or product, e.g. by labelling or stamping the sample or product in question. Alternatively, the information may be remotely, e.g. virtually, associated with the sample or product, e.g. by recording the information in a database or otherwise.

In these embodiments, samples or products for which the associated information indicates that the sample or product has the one or more (particular) properties may be separated from other samples or products (e.g. for which associated information indicates that the sample or product does not have the one or more (particular) properties).

It would alternatively be possible, when it is determined that a food sample has the one or more properties and/or one or more particular properties of interest, to (substantially immediately) separate the sample or product from one or more other samples or products (e.g. which do not have (other than have) the one or more (particular) properties), e.g. without necessary associating information with the sample or product.

The samples or products to be separated may be physically separated from the other samples or products. This may be done, for example, at a downstream location on the production line or slaughter line on which the samples or products are provided. For example, the production line or slaughter line may comprise a forked path, and the samples or products may be separated by means of the forked path. Other arrangements would be possible.

Samples (products) determined not to have the particular properties of interest (e.g. boar taint) may be designated for human consumption, and samples (products) determined to have the particular properties of interest (e.g. boar taint) may be designated for non-human consumption, disposal, or otherwise. It would also or instead be possible to designate samples (products) determined not to have the particular properties of interest (e.g. boar taint) for distribution as a fresh (uncooked) produce, and to designate samples (products) determined to have the particular properties of interest (e.g. boar taint) for use in non-fresh or pre-processed (e.g. cooked) products.

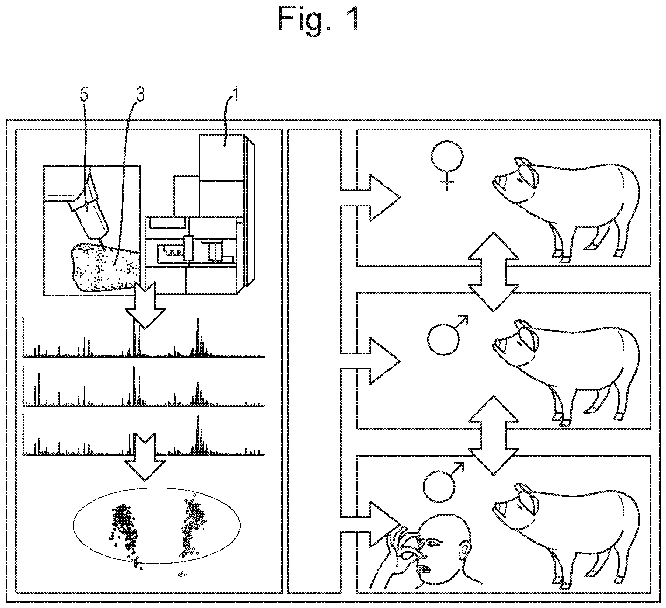

Figure 1 shows schematically an instrumental set up in accordance with an embodiment. In this arrangement, an analytical instrument 1, such as a mass and/or ion mobility spectrometer, is used for the analysis of a porcine sample 3. A probe 5 may be placed upon the sample 3. The probe may be connected to a localised high frequency electric current which may be applied to the surface of the sample 3. The electric current vaporises molecules from the sample 3.

Vapour may travel through a sample inlet tube (not shown) from the probe 5 to the analytical instrument 1. The vapour may be doped with isopropyl alcohol, e.g. which may be provided through the analytical instrument calibration inlet. The doped vapour may then travel through a transfer capillary to a heated impaction surface. lonisation of the doped vapour may occur at the impact surface. The ionised sample may then pass into the ion optics of the analytical instrument for analysis.

As illustrated by Fig. 1, the analytical instrument 1, e.g. mass spectrometer, is configured to produce data which are analysed to determine the presence or absence of boar taint in the sample.

The analytical instrument 1 may comprise any suitable analytical instrument such as for example, a Quadrupole Time of Flight mass spectrometer, a triple Quadrupole mass spectrometer, etc.

Fig. 1 illustrates in particular a probe 5 to which a localised high frequency electric current may be applied. However, other embodiments may use any other device for vaporising the sample 3. Such devices for vaporising the sample 3 may include, for example, a heated probe, a laser irradiating the sample surface, or a spray of a solvent desorbing analyte from the surface, etc.

Fig. 1 illustrates in particular the use of the rapid evaporative ionisation mass spectrometer (REIMS) technique.

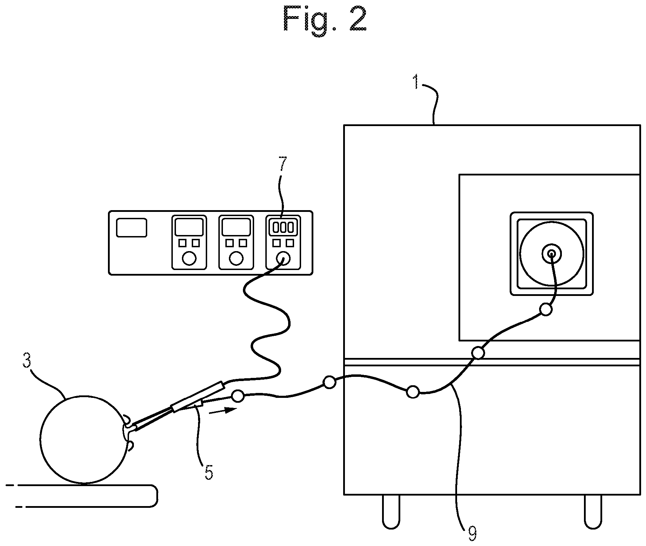

Fig. 2 illustrates the rapid evaporative ionisation mass spectrometer (REIMS) technique according to this embodiment in more detail.

As shown in Fig. 2 a sampling probe 5 may be brought into contact with a sample 3. The sampling probe 5 may comprise, for example, a surgical diathermy device, bipolar forceps or otherwise. An RF voltage from an RF voltage generator 7 may be applied to the sampling probe 5 which causes localised Joule or diathermy heating of the sample 3. As a result, an aerosol, smoke or vapour is generated.

The aerosol, smoke or vapour may then be captured or otherwise aspirated through a port of the sampling probe 5. The aerosol, smoke or vapour may then be passed from the aspiration port of the sampling probe 5 to tubing 9. The tubing 9 may be arranged to transfer the aerosol, smoke or vapour to an atmospheric pressure interface of the analytical instrument 1 (e.g. mass and/or ion mobility spectrometer).

A matrix comprising an organic solvent such as isopropanol may be added to the aerosol, smoke or vapour, e.g. at or prior to the atmospheric pressure interface of the analytical instrument 1. The mixture of aerosol, smoke or vapour and organic solvent may then be arranged to impact upon an optionally heated collision surface. The collision surface may be located within a vacuum chamber of the analytical instrument 1. Alternatively, the collision surface may be arranged external to the analytical instrument 1, e.g. close to the inlet of the analytical instrument 1.

The aerosol, smoke or vapour may be caused to ionise upon impacting the collision surface resulting in the generation of analyte ions. The ionisation efficiency of generating the analyte ions may be improved by the addition of the organic solvent. However, the addition of an organic solvent is not essential.

Figs. 1 and 2 illustrate in particular the use of the rapid evaporative ionisation mass spectrometer (REIMS) technique. However, various other embodiments may use any suitable ambient ionisation technique, e.g. as described above.

For example, the ion source may comprise a desorption electrospray ionisation (DESI) ion source.

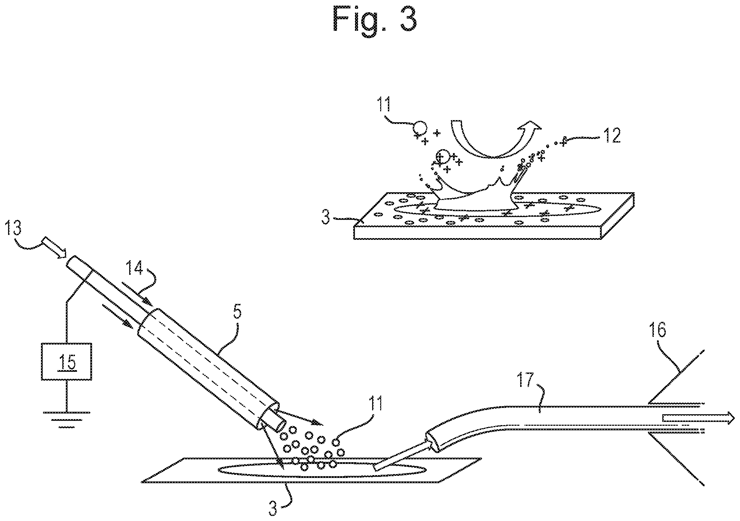

Fig. 3 illustrates the desorption electrospray ionisation (DESI) technique according to an embodiment. The desorption electrospray ionisation (DESI) technique allows for ambient ionisation of samples at atmospheric pressure with little sample preparation.

As shown in Fig. 3, the desorption electrospray ionisation (DESI) technique is an ambient ionisation method that involves directing a spray of (primary) electrically charged droplets 11 directly onto a surface of a sample 3. The electrospray mist is pneumatically directed at the sample by a sampling probe in the form of a sprayer 5 where subsequent ejected (e.g. splashed) (secondary) droplets 12 carry desorbed ionised analytes (e.g. desorbed lipid ions).

The sprayer 5 may be supplied with a solvent 13, nebulising gas 14 such as nitrogen, and voltage from a high voltage (HV) source 15. The solvent 13 may be supplied to a central capillary of the sprayer 5, and the nebulising gas 14 may be supplied to a second capillary that may (at least partially) coaxially surround the central capillary. The arrangement of the capillaries, the flow rate of the solvent 13 and/or the flow rate of the gas 14 may be configured such that solvent droplets are ejected from the sprayer 5. The high voltage may be applied to the central capillary, e.g. such that the ejected solvent droplets 11 are charged.

The charged droplets 11 may be directed at the sample 3 such that subsequent ejected (secondary) droplets 12 carry desorbed analyte ions. The ions travel through air into an atmospheric pressure interface 16 of a mass and/or ion mobility spectrometer or analyser (not shown), e.g. via a transfer capillary 17.

It will be appreciated that numerous other ambient ion sources including those referred to above may be utilised. For example, according to another embodiment the ambient ionisation ion source may comprise a laser ionisation ion source. According to an embodiment the laser ionisation ion source may comprise a mid-IR laser ablation ion source. For example, there are several lasers which emit radiation close to or at 2.94 µm which corresponds with the peak in the water absorption spectrum. The ambient ionisation ion source may comprise a laser ablation ion source having a wavelength close to 2.94 µm, i.e., on the basis of the high absorption coefficient of water at 2.94 µm. According to an embodiment the laser ablation ion source may comprise an Er:YAG laser which emits radiation at 2.94 µm.

Other embodiments are contemplated wherein a mid-infrared optical parametric oscillator ("OPO") may be used to produce a laser ablation ion source having a longer wavelength than 2.94 µm. For example, an Er:YAG pumped ZGP-OPO may be used to produce laser radiation having a wavelength of e.g. 6.1 µm, 6.45 µm or 6.73 µm. In some situations it may be advantageous to use a laser ablation ion source having a shorter or longer wavelength than 2.94 µm since only the surface layers will be ablated and less thermal damage may result. According to an embodiment a Co:MgF2 laser may be used as a laser ablation ion source wherein the laser may be tuned from 1.75-2.5 µm. According to another embodiment an optical parametric oscillator ("OPO") system pumped by a Nd:YAG laser may be used to produce a laser ablation ion source having a wavelength between 2.9-3.1 µm. According to another embodiment a CO2 laser having a wavelength of 10.6 µm may be used to generate the aerosol, smoke or vapour.

The analytical instrument may comprise a control system. The control system may be configured to control the operation of the analytical instrument, e.g. in the manner described herein. The control system may comprise suitable control circuitry that is configured to cause the instrument to operate in the manner of the various embodiments described herein. The control system may also comprise suitable processing circuitry configured to perform any one or more or all of the necessary processing and/or post-processing operations described herein.

The analysis to determine the presence or absence of boar taint in the sample is an untargeted method. An untargeted method is a method that takes into account all or at least a large portion of the data produced, rather than simply looking for an individual indicator. Thus, the analysis of the data is performed using an untargeted approach.

A targeted method (not within the scope of the present invention) should be understood to be a method where a specific marker ion, or a group of specific marker ions, have been identified and the data is mined for this data, to provide the indicator.

The method may be an automated method for testing, identifying and separating animal carcasses that suffer from boar taint from carcasses that do not.

In these embodiments, as a carcass progresses along a slaughter line or otherwise through a production facility or factory, an automated testing mechanism may be arranged to identify the presence and position of a carcass, introduce a sampling probe 5 to the identified carcass, and to vaporise molecules of the sample for analysis.

Sample vapour may then travel through the sample inlet tube. The vapour may be doped with isopropyl alcohol which may be provided through the instrument calibration inlet. The doped vapour may then travel through a transfer capillary 9 to a heated impact surface. Ionisation of the doped vapour may occur at the impact surface. The ionised sample may then pass into the ion optics of the mass spectrometer 1 for analysis.

In accordance with the embodiment shown in Fig. 1, analytical instrument 1 (e.g. mass spectrometer) produces data for analysis to determine the presence or absence of boar taint. This analysis is performed in an untargeted manner. Based on the analysis of the information, the system determines whether the carcass suffers from boar taint, or not.

Upon establishing the presence or absence of boar taint, the carcass may be identified accordingly.

The slaughter line may comprise a forked path for carcasses, e.g. so that carcasses may take plural different paths through the production facility. Carcasses identified as having boar taint may be separated from carcasses free from boar taint by means of the forked path.

A carcass may be stamped or otherwise labelled to identify the presence of absence of boar taint. Such identified carcasses may be separated from other carcasses, e.g. at a later stage in the production line.

After each carcass or set of plural carcasses is tested, the apparatus, e.g. at least the sampling probe 5, may be cleaned. This can help to maintain consistency in the analysis.

One or more sensors may be provided, which is or are configured to sense the position of each carcass, e.g. as it approaches the sampling probe 5. The probe 5 may be configured to automatically (e.g. robotically) move into an appropriate position in order to analyse the sample 3, e.g. by vaporising molecules from the sample 3.

A number of experiments were performed to demonstrate the suitability of the ambient ionisation techniques according to various embodiments such as the REIMS technique for detecting boar taint.

It is believed that boar taint is caused by the build-up of Indole (IND), skatole (SK) and Androsterone (AEON) in the adipose tissue of a boar. IND and SK are two indolic compounds derived from the biological degradation of L-tryptophan in the hindgut and their odour is often described as faecal like. AEON is a pheromone produced in the Leydig cells of the testis and has a urinary or sweaty odour.

Reference standards indole (IND) or 2,3-benzopyrrole, skatole (SK) or 3-methylindole (CAS 83-34-1) and 5α-androst-16-ene-3-one (AEoN) were obtained. For each compound, standard solutions were prepared in isopropyl alcohol at a concentration of 20 µg ml⁻¹. Also a mixture of IND, SK and AEON was prepared in isopropyl alcohol at a concentration of 20 µg ml⁻¹.

Both sow (blank) samples and boar neck fat samples were collected at the slaughter line. In order to select boar samples negative and positive for boar taint, boar carcasses were screened for boar taint at the slaughter line by means of the soldering iron method. All samples were cooled during transport to the laboratory and were immediately stored upon arrival at -80°C until analysis.

The presence or absence of boar taint in the samples was confirmed by an in-house validation using an UHPLC-HR-Orbitrap-MS analysis method. Samples containing levels of IND, SK and/or AEON above and below the odour thresholds (IND: 100 µg kg⁻¹, SK: 200 µg kg⁻¹, AEON: 500 µg kg⁻¹) were considered as positive and negative for boar taint, respectively. In total, 50 samples for each group were collected.

A REIMS hand-held sampling device was used to apply a localised high frequency electric current to the surface of each sample, which vaporises molecules from the latter. The probe had a monopolar cutting device with a shortened knife blade of approximately 6 mm and was applied in a dry cut mode in combination with a diathermy electrosurgical generator at 45 W. Sampling was carried out for 3 to 5 seconds and for each sample, two technical replicates were analysed, thus taking into account repeatability of the analysis. Isopropyl alcohol was used as a dopant to stimulate ionisation of the boar taint compounds.

Mass spectrometric analysis was carried out on a Q-TOF instrument equipped with a helical coiled ribbon collision surface supplied with a constant current power supply set to 4.5A. All analysis occurred in REIMS TOF MS sensitivity mode with continuum data acquisition. Isopropyl alcohol was infused directly into the REIMS source at a constant flow rate of 100 µl min⁻¹ to promote the ionization of lipid (fatty acids and phospholipids) species.

Mass resolution was typically set at 18750 and 19195 for *m*/*z* 281.2537 and 773.5432, respectively. The cone voltage was set at 100 V. Mass spectrometric analysis was performed in negative ionisation mode with a mass range of 50 - 1200 *m*/*z* and scan speed of 1 s/scan. Prior to use, the instrument was calibrated using sodium formate.

For quality control purposes, the endogenous matrix ion PE (34:1) [M-NH4]⁻ C39H76NO8P with *m*/*z* 699.497 was used as a lock-mass compound. Furthermore, 10 replicate burns of a QC sample (bovine muscle) were collected between every 10 pig neck fat samples. The intensity of the base peak ion at *m*/*z* 699.497 was recorded and plotted for quality control monitoring. The probe, transfer tubing and venturi device were cleaned with methanol between every ten samples.

A two-pronged approach was envisaged whereby both targeted and untargeted mass spectrometric analysis were evaluated for the discrimination between boar taint positive and negative carcasses. For targeted analysis purposes, standard solutions (20 µg ml⁻¹) of the boar taint compounds were infused directly into the Xevo G2-XS Q-TOF mass spectrometer to evaluate the ionisation performance of the compounds. Afterwards, the potential of REIMS and influence of the matrix on the ionisation performance was evaluated by soaking blank adipose tissue samples in a standard mixture of the boar taint compounds at a concentration of 10 µg ml⁻¹ prior to analysis.

Untargeted analysis was performed by profiling both boar (negative and positive) and sow (blank) samples and thus effectively providing a mass spectral fingerprint for the latter. Samples were analysed in duplicate on three consecutive days in order to take into account reproducibility. This experiment was repeated on three additional days, with different cone voltage settings (60 or 100 V) of the ionisation source, in order to check the robustness of the measurements. Afterwards, the mass spectrometric fingerprints were used to construct predictive models for the classification of sow and boar samples into blank (sow) and boar taint positive and negative groups.

All data files were pre-processed, including peak alignment and peak picking, using the Progenesis bridge conversion tool. Progenesis QI software was used for lock-mass correction using the endogenous matrix ion 699.4970 and background substraction applying a TIC replicate threshold setting of 100,000. Prior to model building, the data were log-transformed and pareto scaled to generate normally distributed data and reduce noise.

Multivariate regression analysis was performed in SIMCA. Principal component analysis (PCA) was used for unsupervised data analysis to reveal outliers, groups and trends.

Orthogonal partial least-square discriminant analysis was used to construct prediction models to predict the Y-variable (classification of samples in groups) from the X-matrix (mass spectrometric fingerprint). In order to avoid over-fitting of the data, the quality of the orthogonal partial least-square discriminant analysis models was evaluated through the goodness of fit (R²(Y)) and the predictive ability of the models (Q²(Y)). Permutation testing (20 permutations) was performed to assess the risk that the model is spurious, i.e. that the model fits the training set but does not predict Y well for new observations.

Additionally, cross-validated analysis of variance and cross-validation, according to a leave 1/7 out classification, were performed to confirm the validity of the models. In parallel, OMB was used as a model builder recognition software tool. To this end, a linear discriminant analysis (LDA) model including 80% of randomly selected samples of each group was built. The remaining 20% was used as a test set for external validation of the model and run through the recognition software, whereby the observed classifications (based on two burns) were recorded in post-acquisition mode.

To demonstrate the classification potential of REIMS for boar taint, 50 blank (sow), 50 boar taint positive and 50 negative samples were analysed. In a first experiment, both negative and positive ionisation modes were taken into account to increase the range of detected metabolites and considered separately. In negative ionisation mode, better classification accuracy (98%) was observed compared to positive ionization mode (94%). For this reason, it was decided to continue all analysis in negative ionisation mode for final model building.

The PCA plot revealed 17 potential outliers; however, only five of the latter were identified as true suspected outliers using the Hotelling's T2 plot. Four outliers originated from the blank group and one outlier from the boar taint positive group. Since the values of these outliers were located between the 95% and 99% confidence limit, they were omitted from further data analysis.

The validity of the supervised orthogonal partial least-square discriminant analysis model was evaluated through R²(Y) and Q², cross-validated analysis of variance testing and permutation tests. Generally, Q² values > 0.5 are regarded as good for biological models. In these experiments, values obtained for R²(Y) and Q² were 0.872 and 0.756, respectively, indicating an excellent fit and predictive abilities.

Moreover, cross-validated analysis of variance analysis (p < 0.001) demonstrated that the obtained orthogonal partial least-square discriminant analysis model was highly significant.

Finally, permutation testing demonstrated that the predictive abilities of the original model (R²(Y) and Q²(Y)) were higher in comparison to the permutated models.

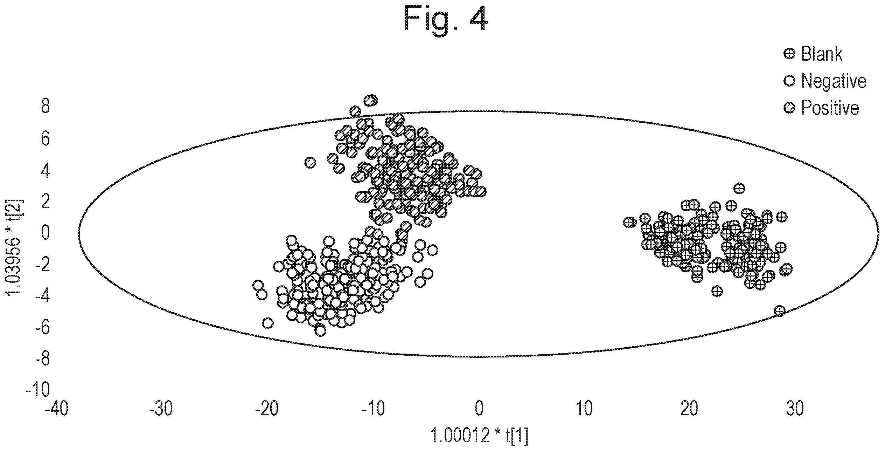

Fig. 4 shows a score plot of a partial least-squares discrimination analysis model for a dataset containing blank (sow) (n=5), negative (untainted) (n=50) and positive (tainted) (n=50) boar neck fat samples in negative ionisation mode. The obtained OPLS-DA model showed separation between the sow and boar groups.

The two boar groups on the other hand showed some overlap. Nevertheless, cross-validation demonstrated that the obtained model had a total correct classification rate of 99% and consequently could be used as a highly accurate predictive tool for the presence of boar taint. All blank and negative samples were correctly classified, whereas of the boar taint positive samples, 98% was correctly classified. The remaining 2% was classified as negative.

The classification results obtained by chemical and sensory analysis, which were used as Y-information for model building, could form the basis of this misclassification. Indeed, based on the sensory scores of the neck fat samples, these samples were severely tainted.

However, chemical analysis by means of UHPLC-HRMS revealed boar taint levels of SK and AEON barely exceeding the proposed odour thresholds of 200 and 500 µg kg⁻¹, respectively. Since previous studies also report a discrepancy between the presence of SK and AEON on the one hand and the sensory evaluation of boar samples on the other, this could potentially lead to biased class information in the Y-axis, causing misclassification in the OPLS-DA model.

In parallel to the orthogonal partial least-square discriminant analysis model, an LDA model including 80% randomly selected samples was built and loaded into a model builder recognition tool. The remaining 20% of samples were run through the real time recognition software, which resulted in a 95% correct classification rate for tainted boar group and thus false negative rate (β error) of ≤ 5%. Additionally, for the sow group a correct classification rate of 95% was observed. For the untainted boar group on the other hand, a correct classification rate of 65% was observed due to the presence of one outlier and allocation of five and one samples as tainted and blank (sow), respectively.

Despite the high percentage of false positive results, a false positive rate (α-error) of ≤ 5% was observed for the sow and untainted boar samples combined. Moreover, in contrast to false negatives, false positive classifications will not result in a loss of consumers' confidence in the porcine meat industry. However, since tainted boar meat is often subject to penalty fees, the number of false positives should be minimised.

Although the indolic compounds are also present, to a lesser extent, in sow carcasses, the risk of the occurrence of boar taint is limited to carcasses of uncastrated pigs. Therefore, only boar carcasses should be screened at the slaughter line.

Therefore, a more simplified orthogonal partial least-square discriminant analysis model was constructed including the boar taint negative and positive group. The obtained model was significant (p < 0.001) and demonstrated excellent predictive properties (R²(Y) = 0.969, Q²(Y) = 0.917).

Moreover, a permutation test showed that the predictive abilities of the more simplified orthogonal partial least-square discriminant analysis model were higher than those obtained for the permutated models.

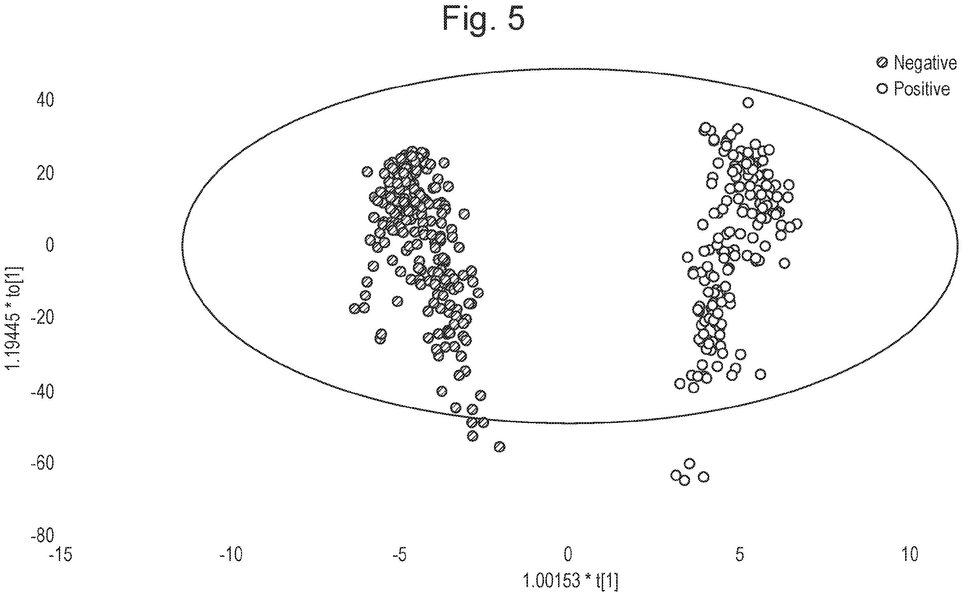

Fig. 5 shows a score plot of a partial least-squares discriminant analysis model for a dataset containing negative (untainted) (n=50) and positive (tainted) (n=50) boar neck fat samples in negative ionisation mode. Cross-validation revealed that all samples were correctly allocated to the boar taint positive or negative group, thus indicating 100% accuracy, specificity and sensitivity of the obtained orthogonal partial least-square discriminant analysis model.

Compared to previous techniques, much higher classification accuracy for tainted and untainted boar carcasses can been attained.

For example, sensitivity and specificity of sensory methods range between 36-88% and 11-85%, respectively, and fluctuate greatly, depending on the trained assessor.

Recently, classification accuracy between tainted and untainted boar samples of 81% was obtained using a portable RAMAN device. However, it should be noted that only true positive and negative samples were taken into account, whereby a cut-off of 1500 µg kg⁻¹ was chosen for AEON, while in the present experiments, a cut-off value of 500 µg kg⁻¹ was considered. Moreover, an uncertainty range of ± 20% of the threshold level was considered for chemical analysis for sample inclusion in the RAMAN experiments.

Furthermore, compared to the targeted detection of IND, SK and AEON, applying an untargeted approach according to various embodiments can improve the true identification of aberrant carcasses. Indeed, up until now, the presence of SK and AEON in neck fat samples of boars only accounts for 76% of the explained variance between the presence of the latter compounds and the intensity of boar taint assessed by trained experts, indicating that also other unknown compounds may contribute to the presence of boar taint.

This was confirmed by a second orthogonal partial least-square discriminant analysis model using quantitative UHPLC-HR-Orbitrap-MS data of IND, SK and AEON as predictive information to classify the samples under investigation as tainted or untainted, as a decrease in accuracy (89%), specificity (82%) and sensitivity (97%) was observed in comparison to the applied untargeted approach (100%).

Consequently, in order to improve correct classify between tainted and untainted boar carcasses, the complete mass spectrum should be taken into account.

To monitor and guarantee the repeatability of the measurements, a QC sample was analysed after every ten pig neck fat samples. Repeatability was then plotted as the intensity of the base peak ion of the endogenous lock-mass compound. In total, 5% and only 0.57% of the measurements exceeded the 2SD and 3SD warning limits, respectively. As over 94% of the measurements lay within these limits, good repeatability was obtained.

In a final experiment, the robustness of REIMS for sample classification was evaluated. To this end, all data were re-acquired on different days but with a change in heater power settings of the collision surface. When taking into account the three sample groups (blank, boar taint negative and positive), a decreased classification accuracy (89%) was observed when working with a lower heater power, whereby an even percentage of false positive and negative results was obtained (16%).

Since ionisation of compounds is enhanced by higher energy and thus a higher heater power, the decrease in accuracy was most likely due to a loss of sensitivity in ion intensity. When omitting the blank group from the model and considering only the two boar groups, excellent classification accuracy (100%) was achieved.

This indicates that despite the change in heater power settings, the REIMS spectra are very reproducible. However, it should be noted that when applying a lower heater power, the obtained orthogonal partial least-square discriminant analysis model showed less reliable predictive abilities as Q²(Y) and R²(Y) were 0.291 and 0.939, respectively, most likely originating from the decrease in sensitivity. This was confirmed in a permutation test, which indicated that the model fits the data well but cannot be used to accurately predict new observations. Consequently, careful consideration should be given to the MS settings in order to ensure the validity of each model.

Untargeted profiling of neck fat samples revealed differences between spectra obtained in negative and positive ionisation mode. Moreover, spectral differences between sow, tainted and untainted boars were observed.

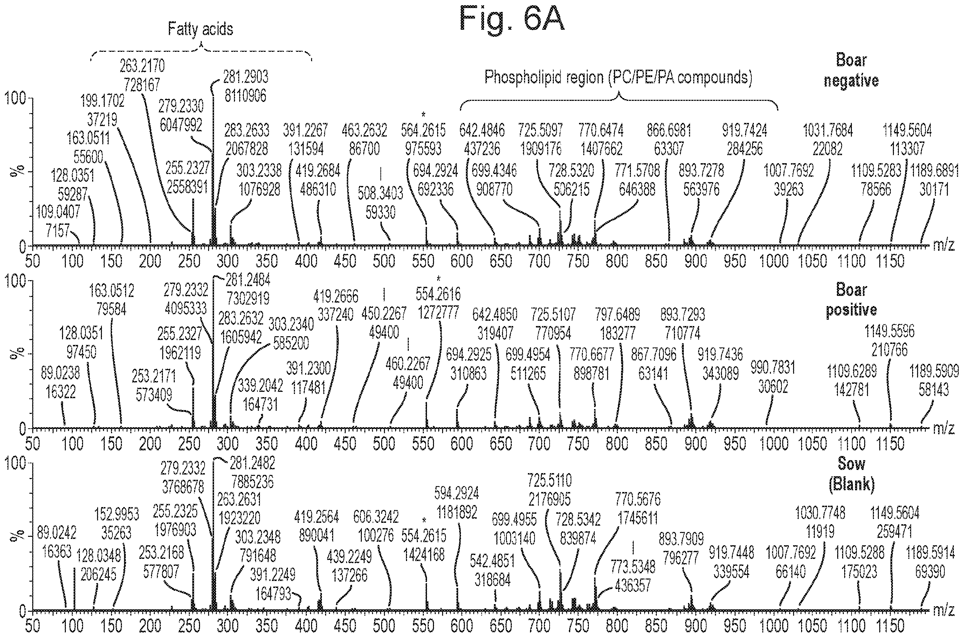

As shown in Fig. 6A, in negative ionisation mode, the most abundant ions were derived from fatty acids, e.g. oleic, linoleic, palmitic and arachidonic acid and phospholipids including phosphatidylethanolamines (PE), phosphatidylcholines (PC) and phosphatidylinositides (PI).

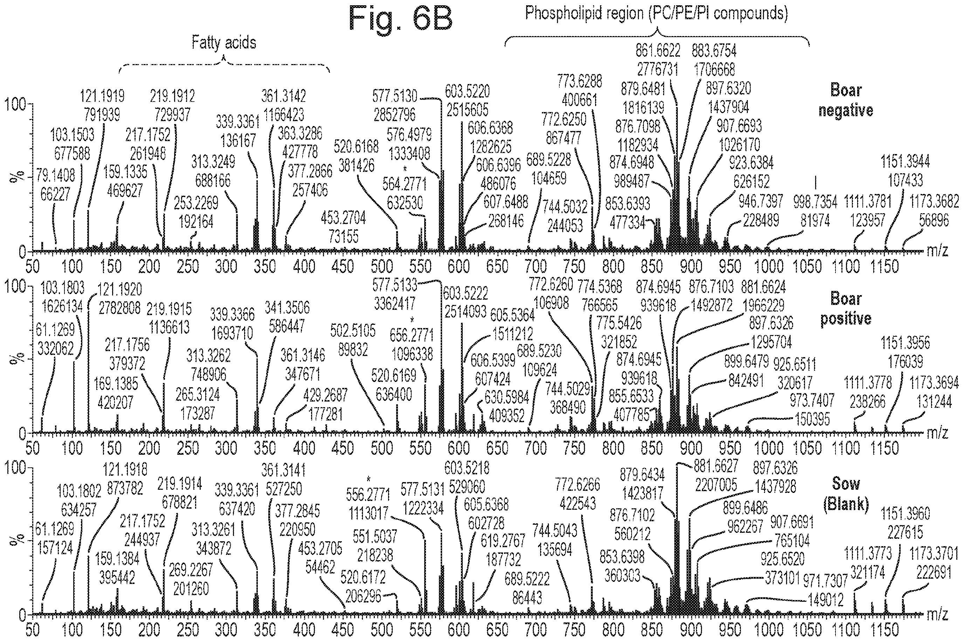

As shown in Fig 6B, in contrast to negative ionisation mode, an increase in the triacylglycerol and phospholipid region of the mass spectra was observed in positive ionisation mode.

In order to situate the spectral differences between the sow, boar taint positive and negative group, putative identification was performed of the most abundant ions in the fatty acid and phospholipid region, providing class information of the compounds. To this end, the selected ions were cross-referenced to the LipidMaps and Lipidblast database. This search was based on the obtained accurate mass and a mass tolerance window of ± 0.01 Da.

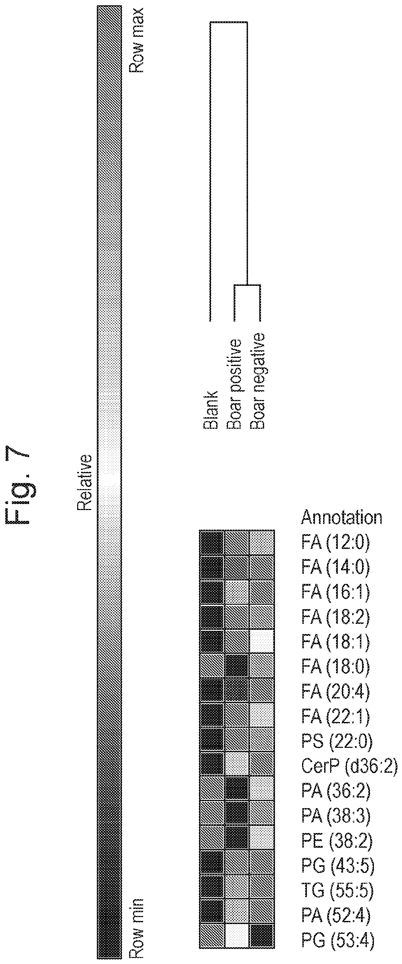

The spectral differences in negative ionisation mode between the sow, boar taint positive and negative group were mainly situated in the fatty acid and phospholipid region of the obtained mass spectra. Moreover, as shown in Fig. 7, primary clustering between the spectra of the boar taint positive and negative group was observed.

As shown in Fig. 7, in general, monounsaturated fatty acids (MUFAs) (16:1, 18:1, 22:1), tentatively identified as palmitoleic acid, oleic acid and erucic acid, respectively, were predominantly present in the boar taint positive group. Intermediate levels were observed in the boar taint negative group and the lowest levels in the blank or sow group. Similarly, also polyunsaturated fatty acids (PUFAs) (18:2 & 20:4), tentatively identified as linoleic acid and arachidonic acid, were mostly abundant in the boar taint positive and negative groups. The saturated fatty acids (SFAs) lauric acid (12:0) and myristic acid (14:0) on the other hand were predominantly present in the boar taint positive and negative groups, respectively. Furthermore, stearic acid (18:0) was mainly present in the blank or sow group in comparison to the two boar groups.

These differences are most likely associated with the differences found in the phospholipid region of the mass spectra as the majority of the signal intensity in the fatty accord region originates from fragmentation of these phospholipids.

Studies have demonstrated similar trends in fatty acid composition in boars, sows and surgically castrated pigs. Significantly lower amounts of SFAs and higher amounts of PUFAs have been found in entire males in comparison to surgically castrated pigs and immunocastrates. A decrease of 21% in n-6-PUFAs in entire males in comparison to boars has been observed.

Furthermore, significantly higher amounts of total PUFAs has been observed in boar carcasses with low levels of AEON (23.4%) in comparison to boar carcasses with high levels of AEON (19.7%). This was due to increased levels of linoleic acid and alpha-linolenic acid. However, higher PUFA and MUFA levels were observed in highly tainted fat samples elsewhere.

The mechanism behind these differences in fatty acid composition lies in the regulation of fat deposition and differences in lipid synthesis and metabolism. Indeed, an increased expression of stearoyl-CoA desaturase and delta-6-desaturase, two enzymes involved in lipid synthesis, has been demonstrated in boar adipose tissue in comparison to castrates.

The latter enzymes are responsible for the formation of unsaturated fatty acids, explaining the higher amount of PUFA found in boars. Not only differences in lipid composition between boars and sows has been observed but also significant reciprocal differences between boars with high and low boar taint levels.

Although the mechanisms behind the influence of high SK and AEON levels on lipid synthesis and metabolism are not completely unravelled, it is believed that that high SK levels induce CYP2E1 activity, an enzyme involved in lipid peroxidation, consequently lowering PUFA levels in adipose tissue.

High levels of AEON on the other hand inhibit gene expression of CYP2E1 and block induction of the latter by SK. Since phospholipids are partly composed out of fatty acids, alterations in fatty acid composition can also manifest them in the phospholipid region. Based on the differences of the latter between sows and boars but more importantly, boars with high and low boar taint levels, lipid profiles may be used to discriminate between these three groups and classify carcasses as tainted or untainted.

It will be appreciated that the Applicants have established that targeted techniques can distinguish between the presence or absence of boar taint in porcine fat samples, e.g. based on the profile of fatty acids and very long chain fatty acids in the 150-500 *m*/*z* region, glycerophospholipids in the 600-900 *m*/*z* region and triglycerides in the 900-1000 *m*/*z* region.

Moreover, the Applicants have demonstrated that tainted carcasses can be correctly classified by an untargeted mass spectrometry approach. This makes REIMS suitable not only for discrimination between gender samples (sow versus boar) but also for discrimination within gender (tainted versus untainted boars).

This discrimination originates from alterations in lipid profiles, mainly situated in the fatty acid and phospholipid region. To this end, a fingerprinting approach is particularly beneficial. Moreover, as REIMS eliminates extensive sample pre-treatment procedures, analysis takes under 10 seconds, which makes it the first technique that enables in-situ detection of boar taint combined with highly accurate classification.

This approach may be implemented in an at-line environment, e.g. using software to enable real time recognition of unknown samples through screening against a known database. For this reason, this new analytical in-situ monitoring platform may be used for other applications in food safety or quality, whereby rapid characterisation of food products is requisite.

In order to demonstrate the benefits of the untargeted approach when compared with conventional targeted approaches, a number of samples of porcine fat were classified using the boar taint model. The fat had been previously characterised using the conventional methods, namely targeted GC-MS (measuring skatole, indole and androstenone levels following sample extraction & concentration) and human sensory analysis. In general, the REIMS results correlated well to the conventional analysis, but matched much more closely to the sensory analysis results, as it is thought that there are other compounds responsible for the odour in addition to the 3 targeted analytes. This shows that the untargeted profiling approach correlates particularly well with sensory analysis.

In addition, the predictive accuracy of the model was determined via independent validation using a selection of 40 previously characterised (using sensory and targeted analysis) samples. The overall predictive accuracy of the model was determined as 90%. This shows a small bias towards taint classification, but it is expected that the false negative rate will be lower than the false positive rate.

Further experiments were performed to demonstrate the effectiveness of the approach according to various embodiments in identifying different sample properties. In particular, REIMS experiments were performed on beef samples to demonstrate that the approach according to various embodiments can be used to distinguish between various beef types.

Three samples were analysed, namely a regular beef rump steak, a premium Welsh beef rump steak, and a buffalo rump steak. Each sample was subjected to twenty 3-5 s burns using the REIMS sampling probe (described above). Data was acquired using a ToF MS in negative ion mode with a scan rate of 1 scan per second over a mass range of 50-1200 m/z.

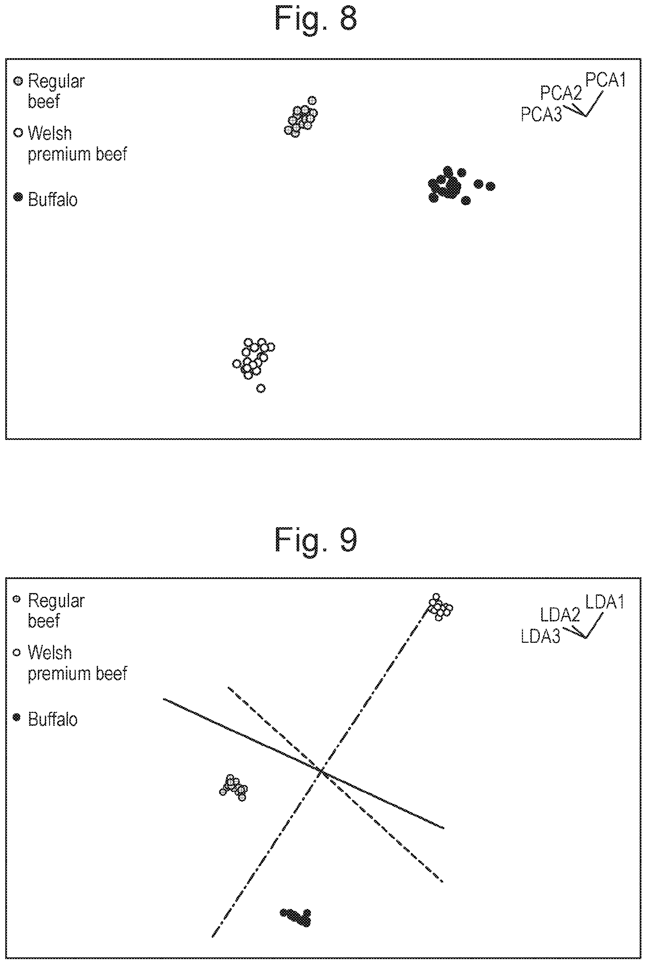

Fig. 8 shows the results of principle component analysis (PCA) of the data sets, and Fig. 9 shows the results of linear discriminant analysis (LCA) of the data sets. It can be seen that the three samples, i.e. a regular beef streak, a premium beef steak and a buffalo steak, can each be separated.

Cross-validation demonstrated that the obtained model had a total correct classification rate of 100% and consequently could be used as a highly accurate predictive tool for the sample type.

Although the present invention has been described with reference to preferred embodiments, it will be understood by those skilled in the art that various changes in form and detail may be made without departing from the scope of the invention as set forth in the accompanying claims.

## Claims

1. A method of analysing a food sample (3) comprising:
generating analyte ions from a food sample (3) using ambient ionisation;
analysing the analyte ions to produce a data set; and
assessing the data set to identify or determine one or more properties of the food sample (3);
wherein the step of assessing the data set to identify or determine one or more properties of the food sample (3) comprises using an untargeted analysis technique;
wherein the step of using an untargeted analysis technique comprises using a predictive model to classify the sample (3) into one of two or more groups; and **characterised in that** the one or more properties comprise one or more organoleptic properties of the food sample (3).

2. The method of claim 1, wherein when the sample (3) is identified or determined to have one or more particular properties, then the method further comprises:
associating with the sample (3) information indicating that the sample (3) has the one or more particular properties; and/or
separating the sample (3) from one or more other samples.

3. The method of any one of the preceding claims, wherein the sample (3) is provided on a production line or a slaughter line.

4. The method of claim 3, wherein when it is determined that the food sample (3) has the one or more particular properties, then the method further comprises:
separating the food sample (3) from one or more other food samples on the production line or slaughter line.

5. The method of any one of the preceding claims, wherein the one or more properties are related to the quality and/or safety of the food sample (3).

6. The method of any one of the preceding claims, wherein the one or more properties comprise:
the presence, quantity and/or identity in the food sample (3) of: (i) one or more microbes; (ii) one or more pathogens; (iii) one or more parasites or excreta or other waste products from parasites; (iv) one or more fungi; (v) one or more xenobiotics; and/or (vi) one or more other contaminants; and/or
the (i) species, subspecies or other taxonomic classification; (ii) gender; (iii) health; (iv) age; and/or (v) geographical origin; of an animal from which the sample is derived; and/or
the quality, taste, composition, flavour or grade of the food sample (3); and/or
the manner in which an animal from which the sample (3) is derived was produced, raised, slaughtered and/or caught.

7. The method of any one of the preceding claims, wherein the sample (3) comprises a porcine meat sample (3), and wherein the step of assessing the data set to identify or determine one or more properties of the food sample (3) comprises assessing the data set to determine whether boar taint is present in the porcine meat sample (3).

8. The method of any one of the preceding claims, wherein the method comprises using a sampling probe (5) to generate aerosol, smoke, vapour, droplets and/or the analyte ions from the sample (3).

9. The method of claim 8, further comprising:
sensing the presence and/or location of the sample (3); and then automatically causing the sampling probe (5) to interact with the sample (3).

10. The method of claim 8 or 9, further comprising causing the sampling probe (5) to interact with the sample (3) for only a few seconds or a few tens of seconds.

11. The method of any one of the preceding claims, wherein generating analyte ions from the sample (3) using ambient ionisation comprises applying an electric current to the sample.

12. The method of any one of the preceding claims, wherein generating analyte ions from the sample (3) using ambient ionisation comprises directing a laser beam onto the sample (3).

13. The method of any one of the preceding claims, wherein generating analyte ions from the sample (3) using ambient ionisation comprises directing a spray of charged droplets (11) onto the sample (3).

14. An analytical instrument (1) for analysing a food sample (3) comprising:
an ambient ionisation system configured to generate analyte ions from a food sample (3);
an analyser configured to analyse the analyte ions to produce a data set; and
processing circuitry configured to assess the data set using an untargeted analysis technique to identify or determine one or more properties of the food sample (3);
wherein the processing circuity is configured to use a predictive model to classify the sample (3) into one of two or more groups; and **characterised in that** the one or more properties comprises one or more organoleptic properties of the food sample (3).

15. A production line or a slaughter line comprising:
the analytical instrument (1) of claim 14;
wherein the production line or slaughter line is configured to: when it is determined that the food product (3) has the one or more particular properties:
associate with the food product (3) information indicating that the food product (3) has the one or more particular properties; and/or
separate the food product (3) from one or more other food products on the production line or slaughter line.

## Patentansprüche

1. Verfahren zum Analysieren einer Lebensmittelprobe (3), umfassend:
Erzeugen von Analytionen aus einer Lebensmittelprobe (3) unter Verwendung einer Umgebungsionisation;
Analysieren der Analytionen zur Herstellung eines Datensatzes; und
Bewerten des Datensatzes zur Identifizierung oder Bestimmung eines oder mehrerer Merkmale der Lebensmittelprobe (3);
wobei der Schritt des Bewertens des Datensatzes zur Identifizierung oder Bestimmung eines oder mehrerer Merkmale der Lebensmittelprobe (3) das Verwenden einer ungezielten Analysetechnik umfasst;
wobei der Schritt des Verwendens einer ungezielten Analysetechnik das Verwenden eines Vorhersagemodells zur Einstufung der Probe (3) in eine von zwei oder mehr Gruppen umfasst; und
**dadurch gekennzeichnet, dass**
das eine oder mehr Merkmale ein oder mehr organoletische Merkmale der Lebensmittelprobe (3) umfassen.

2. Verfahren nach Anspruch 1, wobei, wenn die Probe (3) identifiziert oder bestimmt ist, ein oder mehr besondere Merkmale aufzuweisen, das Verfahren dann weiter umfasst:
Zuweisen von Informationen zu der Probe (3), die darauf hinweisen, dass die Probe (3) das eine oder mehr besonderen Merkmale aufweist; und/ oder
Trennen der Probe (3) von einer oder mehreren anderen Proben.

3. Verfahren nach einem der vorstehenden Ansprüche, wobei die Probe (3) in einer Produktionsstraße oder einer Schlachtstraße bereitgestellt wird.

4. Verfahren nach Anspruch 3, wobei, wenn bestimmt wird, dass die Lebensmittelprobe (3) ein oder mehr besondere Merkmale aufweist, das Verfahren dann weiter umfasst:
Trennen der Lebensmittelprobe (3) von einer oder mehreren anderen Lebensmittelproben in der Produktionsstraße oder der Schlachtstraße.

5. Verfahren nach einem der vorstehenden Ansprüche, wobei sich das eine oder mehrere Merkmale auf die Qualität und/ oder Sicherheit der Lebensmittelprobe (3) beziehen.

6. Verfahren nach einem der vorstehenden Ansprüche, wobei das eine oder mehrere Merkmale umfassen:
das Vorhandensein, Menge und/ oder Identität in der Lebensmittelprobe (3) von: (i) einer oder mehreren Mikroben; (ii) einem oder mehreren Krankheitserregern; (iii) einem oder mehreren Parasiten oder Exkrementen oder anderen Abfallprodukten von Parasiten; (iv) einem oder mehreren Pilzen; (v) einem oder mehreren Fremdstoffen; und/ oder (vi) einem oder mehreren Schadstoffen; und/ oder
die (i) Spezies, Subspezies oder andere taxonomische Einstufung; (ii) Geschlecht; (iii) Gesundheit; (iv) Alter; und/ oder (v) geografische Herkunft; eines Tieres, aus welchem die Probe abgeleitet ist; und/ oder
die Qualität, Geschmack, Zusammensetzung, Geruch oder Grad der Lebensmittelprobe (3);
und/ oder
die Art und Weise, mit der das Tier, aus dem die Probe (3) abgeleitet wird, erzeugt, aufgezogen, geschlachtet und/ oder gefangen wurde.

7. Verfahren nach einem der vorstehenden Ansprüche, wobei die Probe (3) eine Schweinefleischprobe (3) umfasst, und wobei der Schritt des Bewertens des Datensatzes zur Identifizierung oder Bestimmung eines oder mehrerer Merkmale der Lebensmittelprobe (3) das Bewerten des Datensatzes umfasst, um zu bestimmen, ob Wildschweinbeigeschmack in der Schweinefleischprobe (3) vorhanden ist.

8. Verfahren nach einem der vorstehenden Ansprüche, wobei das Verfahren das Verwenden einer Probenentnahmesonde (5) zum Erzeugen von Aerosol, Rauch, Dampf, Tröpfchen und/ oder der Analytionen aus der Probe (3) umfasst.

9. Verfahren nach Anspruch 8, weiter umfassend:
Erfassen des Vorhandenseins und/ oder einer Stelle der Probe (3); und danach automatisch Bewirken, dass die Probenentnahmesonde (5) mit der Probe (3) zusammenwirkt.

10. Verfahren nach Anspruch 8 oder 9, weiter umfassend das Bewirken, dass die Probenentnahmesonde (5) nur einige Sekunden oder einige Zehntelsekunden lang mit der Probe (3) zusammenwirkt.

11. Verfahren nach einem der vorstehenden Ansprüche, wobei das Erzeugen von Analytionen aus der Probe (3) unter Verwendung von Umgebungsionisation das Anlegen eines elektrischen Stroms an die Probe umfasst.

12. Verfahren nach einem der vorstehenden Ansprüche, wobei das Erzeugen von Analytionen aus der Probe (3) unter Verwendung von Umgebungsionisation das Richten eines Laserstrahls auf die Probe (3) umfasst.

13. Verfahren nach einem der vorstehenden Ansprüche, wobei das Erzeugen von Analytionen aus der Probe (3) unter Verwendung von Umgebungsionisation das Richten eines Sprays von geladenen Tröpfchen (11) auf die Probe (3) umfasst.

14. Analyseinstrument (1) zum Analysieren einer Lebensmittelprobe (3), umfassend:
ein Umgebungsionisationssystem, das konfiguriert ist, um Analytionen aus einer Lebensmittelprobe (3) zu erzeugen;
einen Analysator, der konfiguriert ist, um die Analytionen zu analysieren, um einen Datensatz herzustellen;
und
eine Verarbeitungsschaltung, die konfiguriert ist, um den Datensatz unter Verwendung einer ungezielten Analysetechnik zu bewerten, um ein oder mehrere Merkmale der Lebensmittelprobe (3) zu identifizieren oder zu bestimmen;
wobei die Verarbeitungsschaltung konfiguriert ist, um ein Vorhersagemodell zu verwenden, um die Probe (3) in eine von zwei oder mehr Gruppen einzustufen; und
**dadurch gekennzeichnet, dass**
das eine oder mehrere Merkmale ein oder mehrere organoleptische Merkmale der Lebensmittelprobe (3) umfasst.

15. Produktionsstraße oder Schlaschtstraße, umfassend:
ein Analyseinstrument (1) nach Anspruch 14;
wobei die Produktionsstraße oder Schlaschtstraße konfiguriert ist zum: wenn bestimmt wird, dass das Lebensmittelprodukt (3) ein oder mehr besondere Merkmale aufweist:
Zuweisen von Informationen zu dem Lebensmittelprodukt (3), die darauf hinweisen, dass das Lebensmittelprodukt (3) das eine oder mehr besonderen Merkmale aufweist; und/ oder
Trennen des Lebensmittelprodukts (3) von einem oder mehreren Lebensmittelprodukten in der Produktionsstraße oder Schlachtstraße.

## Revendications

1. Procédé d'analyse d'un échantillon alimentaire (3) comprenant :
la génération d'ions analytes depuis un échantillon alimentaire (3) en utilisant une ionisation ambiante ;
l'analyse des ions analytes pour produire un jeu de données ; et
l'évaluation du jeu de données pour identifier ou déterminer une ou plusieurs propriétés de l'échantillon alimentaire (3) ;
dans lequel l'étape de l'évaluation du jeu de données pour identifier ou déterminer une ou plusieurs propriétés de l'échantillon alimentaire (3) comprend l'utilisation d'une technique d'analyse non ciblée ;
dans lequel l'étape de l'utilisation d'une technique d'analyse non ciblée comprend l'utilisation d'un modèle prédictif pour classifier l'échantillon (3) dans l'un parmi deux groupes ou plus ; et
**caractérisé en ce que**
les une ou plusieurs propriétés comprennent une ou plusieurs propriétés organoleptiques de l'échantillon alimentaire (3).

2. Procédé selon la revendication 1, dans lequel, lorsqu'il est identifié ou déterminé que l'échantillon (3) présente une ou plusieurs propriétés particulières, le procédé comprend en outre :
l'association, à l'échantillon (3), d'informations indiquant que l'échantillon (3) présente les une ou plusieurs propriétés particulières ; et/ou
la séparation de l'échantillon (3) d'un ou plusieurs autres échantillons.

3. Procédé selon l'une quelconque des revendications précédentes, dans lequel l'échantillon (3) est prévu sur une chaîne de production ou une chaîne d'abattage.

4. Procédé selon la revendication 3, dans lequel, lorsqu'il est déterminé que l'échantillon alimentaire (3) présente les une ou plusieurs propriétés particulières, le procédé comprend en outre :
la séparation de l'échantillon alimentaire (3) d'un ou plusieurs autres échantillons alimentaires sur la chaîne de production ou la chaîne d'abattage

5. Procédé selon l'une quelconque des revendications précédentes, dans lequel les une ou plusieurs propriétés sont relatives à la qualité et/ou la sécurité de l'échantillon alimentaire (3).

6. Procédé selon l'une quelconque des revendications précédentes, dans lequel les une ou plusieurs propriétés comprennent :
la présence, la quantité et/ou l'identité dans l'échantillon alimentaire (3) de : (i) un ou plusieurs microbes ; (ii) un ou plusieurs pathogènes ; (iii) un ou plusieurs parasites ou excrétas ou autres déchets de parasites ; (iv) un ou plusieurs champignons ; (v) un ou plusieurs xénobiotiques ; et/ou (vi) un ou plusieurs autres contaminants ; et/ou
les (i) espèces, sous-espèces ou autre classification taxonomique ; (ii) genre ; (iii) santé ; (iv) âge ; et/ou (v) origine géographique; d'un animal duquel l'échantillon est dérivé ; et/ou
la qualité, le goût, la composition, l'arôme ou la catégorie de l'échantillon alimentaire (3) ; et/ou
la manière selon laquelle un animal duquel l'échantillon (3) est dérivé a été produit, élevé, abattu et/ou attrapé.

7. Procédé selon l'une quelconque des revendications précédentes, dans lequel l'échantillon (3) comprend un échantillon de viande porcine (3), et dans lequel l'étape de l'évaluation du jeu de données pour identifier ou déterminer une ou plusieurs propriétés de l'échantillon alimentaire (3) comprend l'évaluation du jeu de données pour déterminer si l'odeur sexuelle du verrat est ou non présente dans l'échantillon de viande porcine (3).

8. Procédé selon l'une quelconque des revendications précédentes, dans lequel le procédé comprend l'utilisation d'une sonde d'échantillonnage (5) pour générer un aérosol, une fumée, une vapeur, des gouttelettes et/ou les ions analytes de l'échantillon (3).

9. Procédé selon la revendication 8, comprenant en outre :
la détection de la présence et/ou l'emplacement de l'échantillon (3) ; puis l'instruction automatique à la sonde d'échantillonnage (5) d'interagir avec l'échantillon (3).

10. Procédé selon la revendication 8 ou 9, comprenant en outre l'instruction à la sonde d'échantillonnage (5) d'interagir avec l'échantillon (3) pendant seulement quelques secondes ou quelques dizaines de secondes.

11. Procédé selon l'une quelconque des revendications précédentes, dans lequel la génération d'ions analytes depuis l'échantillon (3) en utilisant une ionisation ambiante comprend l'application d'un courant électrique à l'échantillon.

12. Procédé selon l'une quelconque des revendications précédentes, dans lequel la génération d'ions analytes depuis l'échantillon (3) en utilisant une ionisation ambiante comprend la direction d'un faisceau laser sur l'échantillon (3).

13. Procédé selon l'une quelconque des revendications précédentes, dans lequel la génération d'ions analytes depuis l'échantillon (3) en utilisant une ionisation ambiante comprend la direction d'une pulvérisation de gouttelettes chargées (11) sur l'échantillon (3).

14. Instrument analytique (1) pour analyser un échantillon alimentaire (3) comprenant :
un système d'ionisation ambiante configuré pour générer des ions analytes depuis un échantillon alimentaire (3) ;
un analyseur configuré pour analyser les ions analytes pour produire un jeu de données ; et
une circuiterie de traitement configurée pour évaluer le jeu de données en utilisant une technique d'analyse non ciblée pour identifier ou déterminer une ou plusieurs propriétés de l'échantillon alimentaire (3) ;
dans lequel la circuiterie de traitement est configurée pour utiliser un modèle prédictif pour classifier l'échantillon (3) dans l'un de deux groupes ou plus ; et
**caractérisé en ce que**
les une ou plusieurs propriétés comprennent une ou plusieurs propriétés organoleptiques de l'échantillon alimentaire (3).

15. Chaîne de production ou chaîne d'abattage comprenant :
l'instrument analytique (1) selon la revendication 14 ;
dans laquelle la chaîne de production ou la chaîne d'abattage est configurée pour, lorsqu'il est déterminé que le produit alimentaire (3) présente les une ou plusieurs propriétés particulières :
associer, au produit alimentaire (3), des informations indiquant que le produit alimentaire (3) présente les une ou plusieurs propriétés particulières ; et/ou
séparer le produit alimentaire (3) d'un ou plusieurs autres produits alimentaires sur la chaîne de production ou la chaîne d'abattage.
